# EUROPEAN PATENT APPLICATION

(11) **EP 1 616 948 A1**
(43) Date of publication of application: **18.01.2006**
(21) Application number: 04724433.0
(22) Date of filing: 30.03.2004
(51) Int. Cl.: C12N 15/09, C07K 14/47, C12N 15/11, C12N 1/15, C12N 1/19, C12N 1/21, C12N 5/10, C12P 21/00, A61K 38/17, A61P 35/00

(54) **THIOREDOXIN MODIFICATION**

(30) Priority: 31.03.2003 JP 2003093342; 08.10.2003 JP 2003349109
(71) Applicant: National Institute of Advanced Industrial Science and Technology, Tokyo 100-8921 (JP)
(72) Inventor: ISHII, Yasuyuki, Nat. Inst Adv. Ind. Sc.&Tech, Ikeda-shi, Osaka 5638577 (JP); YODOI, Junji, Nat. Inst. Adv. Ind. Sc.&Tech., Ikeda-shi, Osaka 5638577 (JP); NAKAMURA, Hajime, Hirakata-shi, Osaka 5731118 (JP); KONDO, Norihiko, Nat. Inst. Adv. Ind. Sc.&Tech., Ikeda-shi, Osaka 5638577 (JP)
(74) Representative: Müller-Boré & Partner Patentanwälte
(86) International application number: PCT/JP2004/004523
(87) International publication number: WO 2004/087917

(57) **Abstract**

The present invention relates to a human modified thioredoxin composed of any of the following polypeptides:(a) a polypeptide modified by alteration or chemical modification of a cysteine residue at position 35 with another amino acid in an amino acid sequence of SEQ ID NO:2; and (b) a polypeptide having an amino acid sequence having one or more substituted, deleted, inserted or added amino acids in positions except for positions 32 and 35, preferably positions 32 to 35 in the amino acid sequence of SEQ ID NO:2, and having an apoptosis-inducing activity.

## Description

### TECHNICAL FIELD

The present invention relates to a thioredoxin (referred to as "TRX") modification protein capable of controlling abnormal cell growth and/or cellular function caused by an endogenous and/or exogenous factor in vivo, and an essentially minimized peptide, DNA encoding the modified protein and the essentially minimized peptide, a recombinant expression vector comprising the DNA, a cell transformed with the DNA, methods for producing the modified TRX protein and the essentially minimized peptide, and a pharmaceutical or a pharmaceutical composition comprising the modified TRX protein and/or the essentially minimized peptide.

### BACKGROUND ART

Thioredoxin is a small multifunctional protein of 12 kDa having redox-active disulfide/dithiol in its active site sequence, -Cys-Gly-Pro-Cys- ("Redox regulation of cellular activation" Ann. Rev. Immunol. 1997; 15:351-369.). Thioredoxin has been isolated and identified from various prokaryotic or eukaryotic organisms since it was isolated from *Escherichia coli* as an important enzyme for synthesis of deoxyribonucleotide, a hydrogen donor for ribonucleotide reductase. An adult T cell leukemia-derived factor (ADF) has been first identified by the present inventors as an IL-2 receptor inducing factor produced by T lymphocytes infected with HTLV-1, and is human thioredoxin. Intracellular thioredoxin plays important roles for radical scavenging and controlling transcription factors for redox such as activator protein-1 and nuclear factor-κB ("AP-1 transcriptional activity is regulated by a direct association between thioredoxin and Ref-1" 1997; 94: 3633-3638.). In addition, the human thioredoxin controls signal transduction of p38 mitogen activating protein kinase (MAPK) and apoptosis signal regulating kinase-1 (ASK-1).

Meanwhile, we have reported that the thioredoxin is released out of cells and acts as a cytokine or a chemokine, but its action mechanism has been unknown. It has not been reported that the extracellular TRX is internalized into the cell and that an cell internalization activity which is much higher than that in wild type TRX is produced by modifying a TRX active site.

The present invention analyzes an intracellular controlling activity and a cell internalization activity of TRX, and intends to provide a modified TRX based on the activity and a method for producing the same.

### BRIEF DESCRIPTION OF THE DRAWINGS

FIG. 1 shows results of flow cytometric analysis in Example 2;
FIG. 2 shows results of flow cytometric analysis in Example 2.
FIG. 3 shows results of Western blotting in Example 2.
FIG. 4 shows results of apoptosis analysis in Example 3.
FIG. 5 shows results of measuring uptake amounts of ³H-thymidine using a scintillation counter.

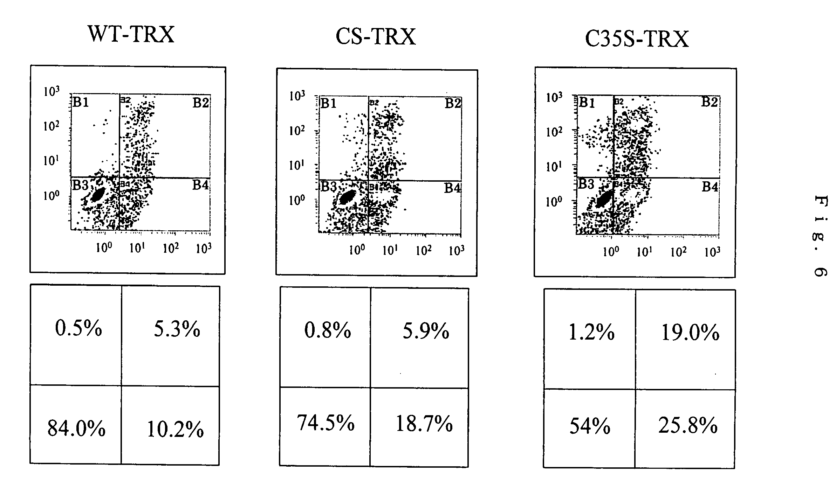

FIG. 6 shows results of enhancing effect-1 on anti-cancer agent action in Example 4. In FIG. 6, CDDP(-) and CDDP(+) indicate the absence and presence of 3 µg/mL cisplatin, respectively. TRX-WT, TRX-CS and TRX-C35S indicate a wild type thioredoxin, a modified thioredoxin of two positions, C32S and C35S, and a modified thioredoxin of C35S, respectively. In CDDP(-), dead cells were increased by about four times in TRX-C35S compared with 4% (TRX-WT) and 3% (TRX-CS). In CDDP(+) (3 µg/mL), the dead cells in TRX-C35S (26%) were increased by 5 or 2 times compared with 5% (TRX-WT) or 13% (TRX-CS).

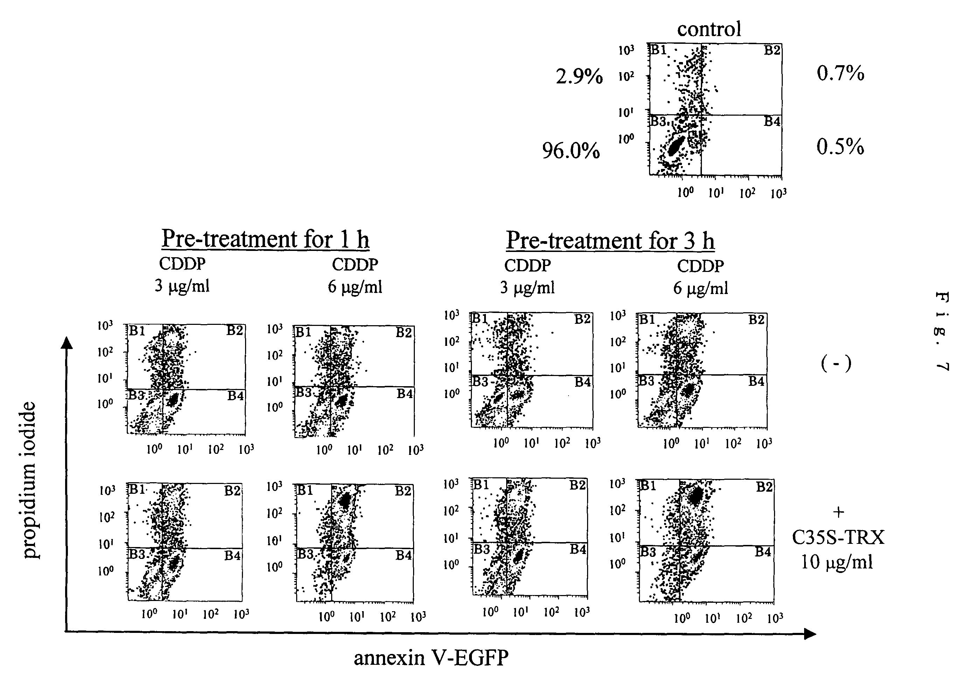

FIG. 7 shows results of enhancing effect-2 on anti-cancer agent action in Example 4. In FIG. 7, CDDP, rec(-) and rec(+) indicate cisplatin, the absence and presence of the recombinant thioredoxin (C35S-TRX), respectively. An upper right quadrant represents double-positive which means a region indicating cell death (region having an anti-cancer effect). For example, when treated with C35S-TRX for one hour, the number of double-positive cells was increased by two times from 10% to 22% in the presence of 10 µg/mL C35S-TRX in CDDP (3 µg/mL). In CDDP (6 µg/mL), the number of the double-positive cells was increased by 4 times from 18% to 76%. Also, when treated with C35S-TRX for one hour, the number of double-positive cells was increased by 4.7 times from 7% to 33% in the presence of 10 µg/mL C35S-TRX in CDDP (3 µg/mL). In CDDP (6 µg/mL), the number of the double-positive cells was increased by 3 times from 23% to 70%.

### DISCLOSURE OF INVENTION

The present inventors analyzed an intracellular controlling activity and a cell internalization activity of TRX in detail, and consequently succeeded in identifying the internalization activity of extracellular human recombinant TRX wild type. Furthermore, they succeeded in producing a modified TRX based on the activity. That is, subjects of the present invention are as follows.
Item 1. A human modified thioredoxin composed of any of the following polypeptides:
   (a) a polypeptide having an amino acid sequence having a substitution of a cysteine residue at position 35 with another amino acid in an amino acid sequence of SEQ ID NO:2;
   (b) a polypeptide having an amino acid sequence having a chemically modified cysteine residue at position 35 in the amino acid sequence of SEQ ID NO:2;
   (c) a polypeptide having an amino acid sequence having one or more substituted, deleted, inserted or added amino acids in positions except for positions 32 and 35, preferably positions 32 to 35 in the amino acid sequence of SEQ ID NO:2, and having an apoptosis-inducing activity; and
   (d) a polypeptide encoded by a DNA encoding a human modified thioredoxin having the substitution of the cysteine residue with another amino acid in the amino acid sequence of SEQ ID NO:2 or a DNA capable of hybridizing with a complementary chain thereof under a stringent condition.
Item 2. The human modified thioredoxin according to Item 1 wherein said another amino acid residue is serine.
Item 3. A gene composed of any of the following DNA and encoding a human modified thioredoxin having an apoptosis-inducing activity:
   (1) a polynucleotide encoding a polypeptide having an amino acid sequence having a substitution of a cysteine residue at position 35 with another amino acid in an amino acid sequence of SEQ ID NO:2 and further having one or more substituted, deleted, inserted or added amino acids in positions except for positions 32 and 35, preferably positions 32 to 35 in the amino acid sequence of SEQ ID NO:2, and having an apoptosis-inducing activity, or a complementary chain thereof; and
   (2) a DNA encoding a human modified thioredoxin having the substitution of the cysteine residue at position 35 with another amino acid in the amino acid sequence of SEQ ID NO:2 or a DNA capable of hybridizing with a complementary chain thereof under a stringent condition and encoding a polypeptide having an apoptosis activity.
Item 4. A recombinant expression vector expressibly incorporating the gene of Item 3.
Item 5. A transformant transformed with the expression vector of Item 4.
Item 6. A method for producing a polypeptide having an apoptosis-inducing activity, the method comprising culturing the transformants of Item 5.
Item 7. A polypeptide for cell internalization of a biologically active substance comprising an amino acid sequence represented by -Cys-Gly-Pro-A, -Cys-Pro-Tyr-A-,-Cys-Pro-His-A- or -Cys-Pro-Pro-A- (A represents any amino acid other than Cys).
Item 8. Use of a polypeptide comprising an amino acid sequence represented by -Cys-Gly-Pro-A, -Cys-Pro-Tyr-A-,-Cys-Pro-His-A- or -Cys-Pro-Pro-A- (A represents any amino acid other than Cys) for cell internalization of a biologically active substance.
Item 9. A method for producing a biologically active substance complex capable of being internalized into cells, the method comprising binding the biologically active substance to a polypeptide comprising an amino acid sequence represented by -Cys-Gly-Pro-A, -Cys-Pro-Tyr-A-,-Cys-Pro-His-A- or -Cys-Pro-Pro-A- (A represents any amino acid other than Cys).
Item 10 A biologically active substance complex capable of being internalized into cells wherein the biologically active substance is bound to a polypeptide comprising an amino acid sequence represented by -Cys-Gly-Pro-A, -Cys-Pro-Tyr-A-,-Cys-Pro-His-A- or -Cys-Pro-Pro-A- (A represents any amino acid other than Cys).
Item 11. The complex according to Item 10 wherein the polypeptide comprising an amino acid sequence represented by -Cys-Gly-Pro-A, -Cys-Pro-Tyr-A-,-Cys-Pro-His-A- or -Cys-Pro-Pro-A- (A represents any amino acid other than Cys) is the polypeptide having an amino acid sequence having a substitution of a cysteine residue at position 35 with another amino acid in an amino acid sequence of SEQ ID NO:2.
Item 12. The complex according to Item 10 or 11 wherein the above biologically active substance is a protein or a polypeptide.
Item 13. A method for producing a polypeptide complex capable of being internalized into cells, the method comprising culturing transformants transformed with a recombinant vector having a polynucleotide encoding the complex in which a biologically active polypeptide has been bound to a polypeptide comprising an amino acid sequence represented by -Cys-Gly-Pro-A, -Cys-Pro-Tyr-A-,-Cys-Pro-His-A- or -Cys-Pro-Pro-A- (A represents any amino acid other than Cys).
Item 14. An anti-cancer agent composed of the modified thioredoxin according to Item 1 or 2.
Item 15. An anti-cancer enhancer composed of the modified thioredoxin of Item 1 or 2.
Item 16. An anti-cancer agent composition comprising the modified thioredoxin of Item 1 or 2 and another anti-cancer agent.
Item 17. A method for treating a cancer, the method comprising administering the modified thioredoxin of Item 1 or 2, if necessary, in combination with another anti-cancer agent to a patient with cancer.
Item 18. A medicine or a pharmaceutical composition comprising the complex of any of Items 10 to 12 and if necessary a pharmaceutically acceptable carrier, excipient or diluting agent.

The present invention will be described in more detail below.

Human thioredoxin (hTRX) herein means a polypeptide composed of 105 amino acids represented by SEQ ID NO:2.

The hTRX of the present invention may be those belonging to thioredoxin superfamily in addition to the human thioredoxin, and those having polypeptides having -Cys-Gly-Pro-Cys-, -Cys-Pro-Tyr-Cys-, -Cys-Pro-His-Cys-, or -Cys-Pro-Pro-Cys- in their active center are exemplified. Among them, the polypeptides having a sequence -Cys-Gly-Pro-Cys- in their active center are preferable.

In a first embodiment of the present invention, it has been found that an apoptosis-inducing activity and a cytostatic activity are expressed by converting a cysteine residue at position 35 into another amino acid to make a modified TRX, which is useful as an anti-cancer agent.

A second embodiment of the invention is based on a finding that cell internalization of the modified TRX is dramatically increased by either converting the cysteine residue into another amino acid to make the modification or chemically modifying the cysteine residue (particularly thiol residue) to make the modified TRX, and it has been first demonstrated that an entity of the cell internalization facilitating effect is based on an amino acid sequence represented by -Cys-Gly-Pro-A, -Cys-Pro-Tyr-A-,-Cys-Pro-His-A- or -Cys-Pro-Pro-A- (A represents any amino acid other than Cys, or chemically modified cysteine).

Cys at position 35 at the C terminal side of the above active center of the TRX may be substituted with any of 19 amino acids (Gly, Ala, Met, Ser, The, Lys, Arg, His, Val, Leu, Ile, Phe, Tyr, Trp, Pro, Gly, Asp, Gln, Asn), and is preferably substituted with Ser.

The Chemically modified cysteine includes those chemically modified in which a thiol (SH) group of cysteine is substituted with a group represented by SR (R is any organic group, which includes, for example, straight or branched alkyl groups having 1 to 18 carbon atoms, straight or branched alkenyl groups having 2 to 18 carbon atoms, straight or branched alkynyl groups having 2 to 18 carbon atoms, straight or branched alkoxyalkyl groups having 2 to 18 carbon atoms, straight or branched hydroxyalkyl groups having 1 to 18 carbon atoms, acyl groups having 1 to 18 carbon atoms, aryl groups having 6 to 18 carbon atoms, aralkyl groups having 7 to 18 carbon atoms and the like, and these groups may be substituted with a substituent such as a halogen atom, hydroxyl, nitro, amino, monoalkylamino, dialkylamino, methoxy, carboxyl or ester group). Such a chemically modified TRX can be easily synthesized by a publicly known method.

The preferable modified thioredoxin and the gene encoding the modified thioredoxin of the present invention is shown in SEQ ID NO:11.

It has been found that the modified thioredoxin of the invention expresses the apoptosis-inducing activity and the cytostatic activity and is useful as the anti-cancer agent.

Furthermore, the modified thioredoxin of the invention can enhance the anti-cancer effect by combining with another anti-cancer agent. In particular, the modified thioredoxin can induce the anti-cancer effect, enhance the effect of the anti-cancer agent, and reduce side effects by combining with the anti-cancer agent at a concentration less than an effective amount for anti-cancer.

The anti-cancer agents whose effect is enhanced by the combination include adriamycin, methotrexate, taxol, 5-fluorouracil, vinblastine, vincristine, mitomycin, cisplatin, daunomycin, etoposide, taxotere, and the like.

The modified thioredoxin of the present invention and the anti-cancer agent may be concurrently administered or may be separately administered.

One preferable embodiment of the invention relates to a method for treating a cancer wherein the modified thioredoxin and the anti-cancer agent are administered.

The cancers capable of being treated are not particularly limited, and include stomach cancer, colon cancer, rectal cancer, hepatic cancer, gall bladder/bile duct cancer, pancreatic cancer, lung cancer, breast cancer, urinary bladder cancer, cervical cancer and the like.

A therapeutically effective amount of the anti-cancer agent composed of the modified thioredoxin of the present invention is about 0.01 to 100 mg per adult patient with cancer daily, and the therapeutically effective amount when used as an anti-cancer enhancer is about 0.001 to 10 mg.

In one preferable embodiment of the present invention, the modified thioredoxin of the invention can be produced based on the human thioredoxin of SEQ ID NO:2 by a gene-engineering technique known publicly. The modification has one or more substituted, deleted, added or inserted amino acids at positions except for the positions 32 and 35, preferably the positions 32 to 35 in the amino acid sequence of SEQ ID NO:2, and has the apoptosis-inducing activity. Such mutations (substitution, deletion, addition and insertion) include artificial mutations in addition to the naturally occurring mutations (e.g., alleles). A procedure for causing the artificial mutation can include, but is not limited to, site-directed mutagenesis (Nucleic Acids Res. 10, 6487-6500, 1982) and the like. The number of mutated amino acids is not limited as long as the apoptosis-inducing activity is not lost, but is preferably within 20 amino acids, more preferably within 15 amino acids, still more preferably within 10 amino acids and most preferably within 5 amino acids.

In another preferable embodiment of the present invention, a DNA encoding a human modified thioredoxin having a substitution of a cysteine residue at position 35 with another amino acid in an amino acid sequence of SEQ ID NO:2 or a DNA capable of hybridizing with a complementary chain thereof under a stringent condition and encoding a polypeptide having an apoptosis activity is included, and a protein encoded by the DNA has the apoptosis-inducing activity.

The "stringent condition" herein refers to the condition where only the specific hybridization takes place whereas no non-specific hybridization takes place. Such a condition is typically concentrations equivalent to about "1 x SSC and 0.1% SDS at 37°C", preferably about "0.5 x SSC and 0.1% SDS at 42°C", and more preferably "0.2 x SSC and 0.1% SDS at 65°C". The DNA of the present invention typically has high homology to the DNA (e.g., DNA described in SEQ ID NO:11) encoding the polypeptide of the invention where the position 35 has been mutated. The high homology indicates the homology of 75% or more, preferably 90% or more, more preferably 95% or more and particularly 99% or more.

The protein of the present invention can be obtained by incorporating the gene of the invention described later into an expression vector and expressing in appropriate host cells. As the vector, it is possible to appropriately select and use those known publicly, e.g., pTrc-HisA and the like. The host cells include eukaryotic cells such as mammalian cells and yeast cells and prokaryotic cells such as cells from *Escherichia coli, Bacillus subtilis,* algae and fungi, and any of them may be used.

Without intending to be limited by theory, it is believed that the modified TRX expresses the apoptosis-inducing activity or the cytostatic activity because the modified TRX functions as an antagonist of the TRX when Cys at position 35 is substituted with another amino acid such as Ser.

Furthermore, it is believed that the modified TRX having the substitution at position 35 with another amino acid such as Ser is bound to a cell surface at Cys of position 32, no binding to the cell surface occurs at position 35 because of no Cys at position 35 and consequently the modified TRX is rapidly internalized in to the cell. This mechanism is also supported by the fact that all of a modified TRX of Ser³² (Cys at position 35; SEQ ID N0:14), a modified TRX of Ser³²Ser³⁵ (SEQ ID NO:13) and the wild type TRX (Cys at positions 32 and 35; SEQ ID NO:2) are scarcely internalized into the cell.

That is, it is obvious that the amino acid sequence represented by Cys-Gly-Pro-A, -Cys-Pro-Tyr-A-,-Cys-Pro-His-A- or -Cys-Pro-Pro-A- (A represents any amino acid other than Cys), particularly Cys-Gly-Pro-A (A is the same as the above) plays a role of a leader peptide to internalize a biologically active substance into the cells, and it is possible to internalize the biologically active substance which has been hardly internalized into the cells by binding the tetrapeptide to the biologically active substance.

The "modified one" herein is used as a term which encompasses both a variant and a modification.

The biologically active substances include pharmaceutical compounds which are internalized to function, oligopeptides, polypeptides, monosaccharides, disaccharides or polysaccharides, lipids and the like, and preferably pharmaceutical compounds, oligopeptides and polypeptides (including glycoproteins) are exemplified. It is also possible to enhance selectivity for a target cell by binding an appropriate sequence to the N and C termini of the leader peptide comprising the above tetrapeptide.

For the pharmaceutical compounds as the biologically active substances, anti-cancer agents, anti-viral agents and the like are exemplified. As biologically active peptides, enzymes, antibodies, hormones and the like are exemplified.

The leader peptide of the present invention can bind the biologically active substance through a spacer peptide if necessary. For example, when the biologically active substance is a polypeptide, the above leader peptide is bound to the biologically active polypeptide through the spacer peptide to make a complex, and it is possible to produce the complex by introducing a DNA encoding the complex into an appropriate recombinant vector, transforming a host of bacteria such as *Escherichia coli,* animal cells such as CHO, or yeast, and culturing the transformant.

By introducing an intracellularly cleavable short chain peptide as the spacer, it is possible to intracellularly lead to the biologically active substance, particularly the biologically active oligopeptide or polypeptide. Such a cleavable peptide includes -AYVHDAPVK- which is the sequence of a cleaved site of human pro-interleukin-1B (pro-IL-1β) specifically cleaved by caspase-1 and -GDEVDGVK- which is the sequence of a cleaved site of human poly-ADP-ribose polymerase (PARP) cleaved by caspase-3 and -7 and so on.

### BEST MODE FOR CARRYING OUT THE INVENTION

The present invention will be described in more detail with reference to the following Examples, but these Examples are for illustrating and do not limit the scope of the invention.

### [Example 1]

### Production of recombinant TRX wild type and modified TRX

### 1. Construction of Escherichia coli (E. coli) expression system

Cysteine residues at positions 32 and/or 35 present in the TRX polypeptide were substituted with serine residues by replacing nucleotides at the positions in the TRX gene of SEQ ID NO:1. A variant gene (TRX-C32S; SEQ ID NO:14) having the serine residue instead of the cysteine residue at position 32 was made by PCR with a plasmid DNA where the human TRX cDNA (SEQ ID NO:1) had been inserted into a vector pcDNA3.1 as a template using primers 5'-GGA TCC GTG AAG CAG ATC GAG AGC AAG-3' (SEQ ID NO:3) and 5'-CTT GAT CAT TTT GCA AGG CCC AGA CCA-3' (SEQ ID NO:5) as well as primers 5'-GTC GAC TTA GAC TAA TTC ATT AAT GGT GGC-3' (SEQ ID NO:4) and 5'-TGG TCT GGG CCT TGC AAA ATG ATC AAG-3' (SEQ ID NO:6). Subsequently, amplified two DNA fragments were mixed at an equal amount, further the primers 5'-GGA TCC GTG AAG CAG ATC GAG AGC AAG-3' (SEQ ID NO:3) and 5'-GTC GAC TTA GAC TAA TTC ATT AAT GGT GGC-3' (SEQ ID NO:4) were added, and then a full length of TRX-C32S was amplified by PCR. The PCR was performed under a cycle condition at 95°C for one minutes, 56°C for one minutes for annealing and 72°C for two minutes for extension. Likewise, a variant gene (TRX-C35S; SEQ ID NO:12) having the serine residue instead of the cysteine residue at position 35 in TRX or a variant gene (TRX-C32S/C35S; SEQ ID NO:13) having the serine residues instead of the cysteine residues at positions 32 and 35 in TRX were made by PCR using the primers 5'-CTT GAT CAT TTT GGA AGG CCC ACA CCA-3' (SEQ ID NO:7) and 5'-TGG TGT GGG CCT TCC AAA ATG ATC AAG-3' (SEQ ID NO:8) or 5'-TGG TCT GGG CCT TCC AAA ATG ATC AAG-3' (SEQ ID NO:9) and 5'-CTT GAT CAT TTT GGA AGG CCC AGA CCA-3' (SEQ ID NO:10) instead of the primers used for making the TRX-C32S gene. The wild type TRX (TRX-WT)was obtained by amplifying the full length of TRX-WT by PCR with the plasmid DNA where the human TRX cDNA (SEQ ID NO:1) had been inserted into the vector pcDNA3.1 as the template DNA using primers 5'-GGA TCC GTG AAG CAG ATC GAG AGC AAG-3' (SEQ ID NO:3) and 5'-GTC GAC TTA GAC TAA TTC ATT AAT GGT GGC-3' (SEQ ID NO:4). The DNA fragment of TRX-WT, TRX-C32S, TRX-C35S or TRX-C32S/C35S amplified by PCR was ligated to a TOPO cloning vector (supplied from Invitrogen) and subsequently introduced into *E*. *coli* host cells. A plasmid DNA was collected from a transformed clone, and the sequence of an inserted DNA was identified by DNA sequencing. Subsequently, the plasmid DNA was cleaved with restriction enzymes, BamHI and SalI, the resulting fragment was ligated to a recombinant protein expression vector pQE80L (supplied from Qiagen) added a histidine tag, and then the *E. coli* host cells, XL-1 Blue were transformed.

### 2. Culture of E. coli cells which produce recombinant TRX wild type or modified TRX

*E. coli* cells transformed with the plasmid DNA pQE80L in which the TRX wild type gene or the modified TRX gene had been inserted were seeded in 3L of terrific broth (BRL) (containing 100 µg/mL of ampicillin) after preculture, and cultured for 4 hours. Subsequently, IPTG was added at a final concentration of 1 mM, and the culture was continued for additional 2 to 4 hours.

### 3. Purification of recombinant TRX wild type and modified TRX

Collected cells were suspended in lysis buffer (protease inhibitor, 0.8 mM Imidazol, 2-mercapt ethanol) containing 2 mM lysozyme, and disrupted by a sonicator. After centrifuging at 15,000 rpm for 30 minutes, a supernatant was collected, and applied on an Ni agarose column (supplied from Qiagen) equilibrated with PBS (Ni column had been previously replaced with PBS). After applying the sample, the column was washed with 20 mM imidazol-containing PBS, and the sample was eluted with 80 mM imidazol-containing PBS. The eluted sample solution was replaced with the PBS solution using a PD-10 column.

### [Example 2]

### Internalization of recombinant TRX wild type or modified TRX into cultured cell

### 1. Binding capacity to cells

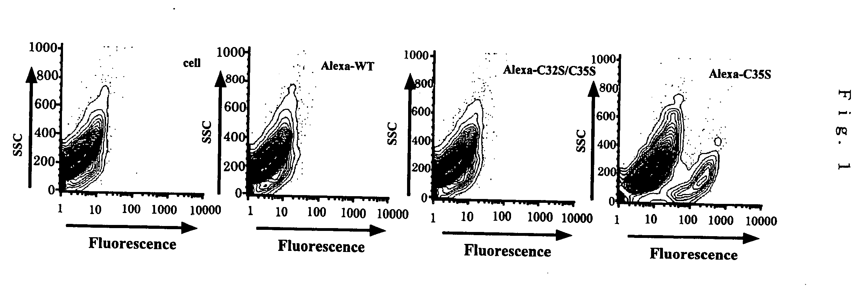

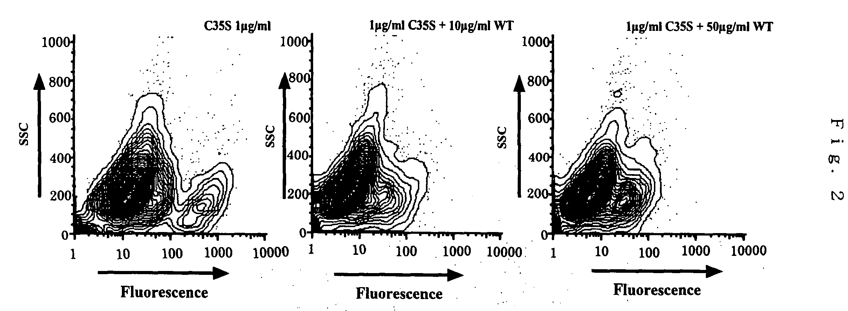

Fluorescently labeled TRX-WT, TRX-C35S or TRX-C32S/C35S at a final concentration of 1 µg/ml was added to 5 x 10⁵ cells of an HTLV-1 infected human T cell line, ATL2, incubated at 4°C for 30 minutes, and then washed with buffer (0.1% sodium azide-containing phosphate buffer) for flow cytometry. Analysis by the flow cytometry was performed (FIG. 1). As a result, it was identified that only the TRX-C35S could be bound to the cells. It was also identified that this binding was inhibited in the coexistence of the TRX-WT in a largely excessive amount (FIG. 2).

### 2. cell internalization

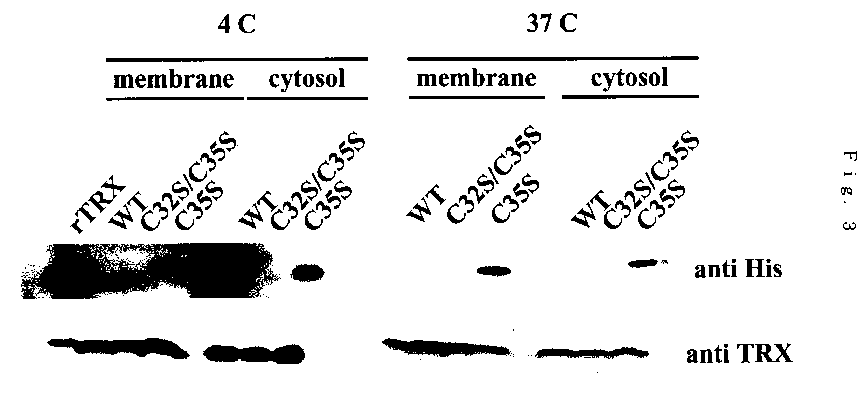

The histidine-tagged recombinant protein, TRX-WT, TRX-C35S or TRX-C32S/C35S was added at a final concentration of 10 µg/ml to ATL2 cells, and incubated at 4°C or 37°C for one hour. Subsequently, the cells were collected, washed with the phosphate buffer, then, 1 x 10⁷ cells were suspended in hypotonic buffer, and disrupted by a nitrogen gas cell homogenizer (suppled from Pearl). After centrifuging at 1,000 g, the supernatant was collected, and a cytosol fraction of the supernatant was collected by centrifugation at 10,000 g. The cytosol fraction was analyzed by Western blotting using SDS-polyacrylamide gel electrophoresis and an anti-TRX monoclonal antibody (FIG. 3). As a result, it was identified that only the TRX-C35S was detected in the cytosol fraction.

### [Example 3]

### Biological activity of TRX wild type or modified TRX

### 1. Apoptosis-inducing activity

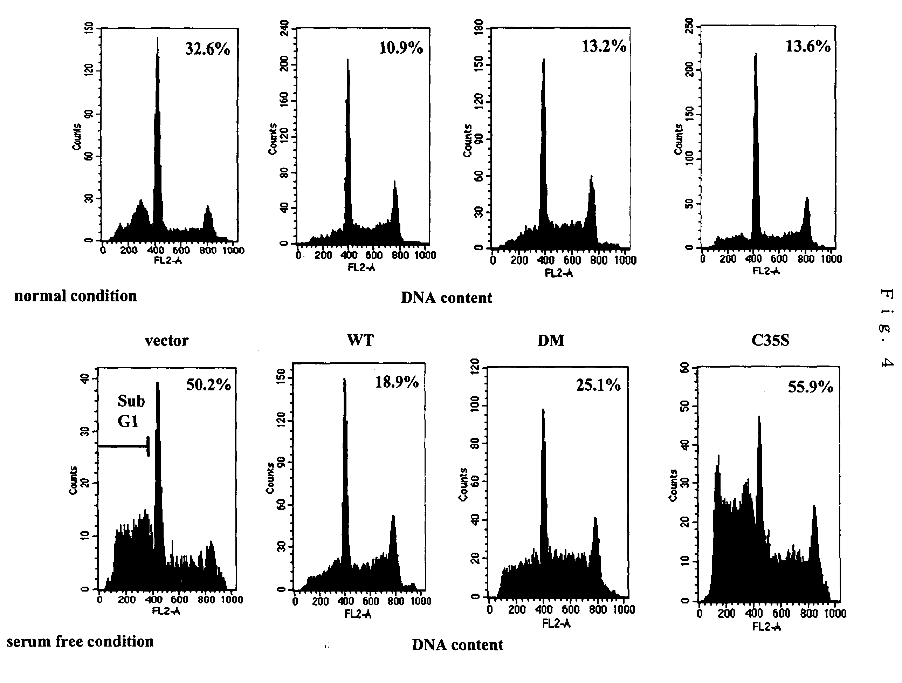

A human T cell line, Jurkat into which the TRX-WT, TRX-C35S or TRX-C32S/C35S gene had been introduced was cultured in serum free RPMI medium for 72 hours to analyze induced apoptosis (FIG. 4). As a result, the apoptosis was further facilitated in Jurkat cells with intracellular high expression of the TRX-C35S compared with Jurkat cells to which the TRX-WT or TRX-C32S/C35S had been introduced.

### 2. Cytostatic activity

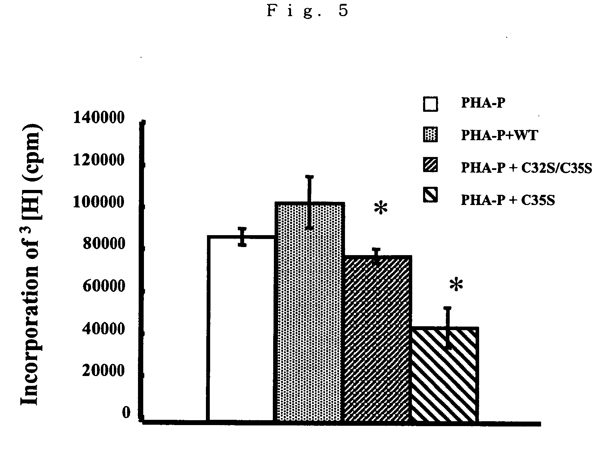

Human peripheral blood mononuclear cells at 5 x 10⁵ were suspended in 1 ml of 10% fetal calf serum-containing RPMI medium, then PHA at a final concentration of 28 ng/mL and 10 µg/mL of the TRX-WT, TRX-C32S/C35S or TRX-C35S were added and cultured. After 96 hours, ³H-thymidine was added and uptake amounts thereof was measured by a scintillation counter (FIG. 5). As a result, it was identified that cell proliferative capacity was increased in the cells with the recombinant TRX-WT protein whereas the cell proliferation was inhibited in the cells with the recombinant TRX-C35S protein, compared to the cells with no TRX.

### [Example 4]

### 1. enhancing effect of anti-cancer agent behavior 1

Cisplatin (CDDP; cis-platinum (II)-diammine dichloride, Sigma) at a final concentration of 3 µg/mL was added to cells with high expression of the TRX gene, which were established by introducing the wild type (WT)TRX, C32S/C35S derivative (CS)TRX or C35S derivative-TRX gene into the human T cell line, Jurkat. Annexin V-FITC and propidium iodide(Medical & Biological Laboratories CO., LTD) were bound to the cells after culturing for 24 hours, and the numbers of double-positive cells which exhibited the apoptosis were analyzed by the flow cytometer. As a result, it was identified that the number of the dead cells which were double-positive for Annexin V-FITC and propidium iodide in the cell line which expressed the C35S-TRX at a high level was increased by about 4 times compared to the cell line which expressed the WT-TRX or the CA-TRX.

### 2. enhancing effect of anti-cancer agent behavior 2

The recombinant C35S-TRX protein was added at a concentration of 10 µg/mL into the culture of the human T cell line, Jurkat, incubated for one hour or three hours, and then cisplatin was added at a final concentration of 3 µg/mL or 6 µg/mL. Annexin V-FITC and propidium iodide were bound to the cells after culturing for 24 hours, and the numbers of double-positive cells which exhibited the apoptosis were analyzed by the flow cytometer. As a result, in the groups to which the recombinant C35S-TRX protein had been previously added, the number of double-positive cells for Annexin V-FITC and propidium iodide was increased compared to the groups to which no recombinant protein had been added, and increased by about 2 times in the group with 3 µg/mL of cisplatin and by about 4 times in the group with 6 µg/mL of cisplatin. The effect was higher in the group pretreated with the C35S-TRX protein for 3 hours than the group pretreated for one hour.

As in the above, the preferable embodiments of the present invention have been described, and it will be understood by those skilled in the art that various changes and improvements can be made without departing from the spirit of the invention. Therefore, the scope of the invention should be determined only by the following claims.

According to the present invention, it becomes possible to produce the modified TRX protein having the high cytostatic activity stably and on a large scale and develop the vector which takes advantage of the peptide sequences derived from the modified TRX capable of being rapidly internalized into the cells. The biologically active substance (proteins, lipids, nucleic acids, organic compounds, inorganic compounds) fused with the modified TRX protein or the peptide derived from the modified TRX can be utilized for the treatment and/or prophylaxis in various disease fields.

## Claims

1. A human modified thioredoxin composed of any of the following polypeptides:
(a) a polypeptide having an amino acid sequence having a substitution of a cysteine residue at position 35 with another amino acid in an amino acid sequence of SEQ ID NO:2;
(b) a polypeptide having an amino acid sequence having a chemically modified cysteine residue at position 35 in the amino acid sequence of SEQ ID NO:2;
(c) a polypeptide having an amino acid sequence having one or more substituted, deleted, inserted or added amino acids in positions except for positions 32 and 35, preferably positions 32 to 35 in the amino acid sequence of SEQ ID NO:2, and having an apoptosis-inducing activity; and
(d) a polypeptide encoded by a DNA encoding a human modified thioredoxin having the substitution of the cysteine residue with another amino acid in the amino acid sequence of SEQ ID NO:2 or a DNA capable of hybridizing with a complementary chain thereof under a stringent condition.

2. The human modified thioredoxin according to claim 1 wherein said another amino acid residue is serine.

3. A gene composed of any of the following DNA and encoding a human modified thioredoxin having an apoptosis-inducing activity:
(1) a polynucleotide encoding a polypeptide having an amino acid sequence having a substitution of a cysteine residue at position 35 with another amino acid in an amino acid sequence of SEQ ID NO:2 and further having one or more substituted, deleted, inserted or added amino acids in positions except for positions 32 and 35, preferably positions 32 to 35 in the amino acid sequence of SEQ ID NO:2, and having an apoptosis-inducing activity, or a complementary chain thereof; and
(2) a DNA encoding a human modified thioredoxin having the substitution of the cysteine residue at position 35 with another amino acid in the amino acid sequence of SEQ ID NO:2 or a DNA capable of hybridizing with a complementary chain thereof under a stringent condition and encoding a polypeptide having an apoptosis activity.

4. A recombinant expression vector expressibly incorporating the gene of claim 3.

5. A transformant transformed with the expression vector of claim 4.

6. A method for producing a polypeptide having an apoptosis-inducing activity, the method comprising culturing the transformants of claim 5.

7. A polypeptide for cell internalization of a biologically active substance comprising an amino acid sequence represented by -Cys-Gly-Pro-A, -Cys-Pro-Tyr-A-,-Cys-Pro-His-A- or -Cys-Pro-Pro-A- (A represents any amino acid other than Cys).

8. Use of a polypeptide comprising an amino acid sequence represented by -Cys-Gly-Pro-A, -Cys-Pro-Tyr-A-,-Cys-Pro-His-A- or -Cys-Pro-Pro-A- (A represents any amino acid other than Cys) for cell internalization of a biologically active substance.

9. A method for producing a biologically active substance complex capable of being internalized into cells, the method comprising binding the biologically active substance to a polypeptide comprising an amino acid sequence represented by -Cys-Gly-Pro-A, -Cys-Pro-Tyr-A-,-Cys-Pro-His-A- or -Cys-Pro-Pro-A- (A represents any amino acid other than Cys).

10. A biologically active substance complex capable of being internalized into cells wherein the biologically active substance is bound to a polypeptide comprising an amino acid sequence represented by -Cys-Gly-Pro-A, -Cys-Pro-Tyr-A-,-Cys-Pro-His-A- or -Cys-Pro-Pro-A- (A represents any amino acid other than Cys).

11. The complex according to claim 10 wherein the polypeptide comprising an amino acid sequence represented by -Cys-Gly-Pro-A, -Cys-Pro-Tyr-A-, -Cys-Pro-His-A- or -Cys-Pro-Pro-A- (A represents any amino acid other than Cys) is the polypeptide having an amino acid sequence having a substitution of a cysteine residue at position 35 with another amino acid in an amino acid sequence of SEQ ID NO:2.

12. The complex according to claim 10 or 11 wherein the above biologically active substance is a protein or a polypeptide.

13. A method for producing a polypeptide complex capable of being internalized into cells, the method comprising culturing transformants transformed with a recombinant vector having a polynucleotide encoding the complex in which a biologically active polypeptide has been bound to a polypeptide comprising an amino acid sequence represented by -Cys-Gly-Pro-A, -Cys-Pro-Tyr-A-,-Cys-Pro-His-A- or -Cys-Pro-Pro-A- (A represents any amino acid other than Cys).

14. An anti-cancer agent composed of the modified thioredoxin of claim 1 or 2.

15. An anti-cancer enhancer composed of the modified thioredoxin of claim 1 or 2.

16. An anti-cancer agent composition comprising the modified thioredoxin of claim 1 or 2 and another anti-cancer agent.

17. A method for treating a cancer, the method comprising administering the modified thioredoxin of claim 1 or 2, if necessary, in combination with another anti-cancer agent to a patient with cancer.

18. A medicine or a pharmaceutical composition comprising the complex of any of claims 10 to 12 and if necessary a pharmaceutically acceptable carrier, excipient or diluting agent.


SEQUENCE LISTING

<110> NATIONAL INSTITUTE OF ADVANCED INDUSTRIAL SCIENCE AND TECHNOLOGY

<120> Thioredoxin derivatives

<130> P04-01

<160> 14

<170> PatentIn version 3.1

<210> 1
<211> 318
<212> DNA
<213> Homo sapiens

<220>
<221> CDS
<222> (1)..(318)
<223>

<400> 1
atg gtg aag cag atc gag agc aag act gct ttt cag gaa gcc ttg gac 48
Met Val Lys Gln Ile Glu Ser Lys Thr Ala Phe Gln Glu Ala Leu Asp
1               5                   10                  15

gct gca ggt gat aaa ctt gta gta gtt gac ttc tca gcc acg tgg tgt 96
Ala Ala Gly Asp Lys Leu Val Val Val Asp Phe Ser Ala Thr Trp Cys
            20                  25                  30

ggg cct tgc aaa atg atc aag cct ttc ttt cat tcc ctc tct gaa aag 144
Gly Pro Cys Lys Met Ile Lys Pro Phe Phe His Ser Leu Ser Glu Lys
        35                  40                  45

tat tcc aac gtg ata ttc ctt gaa gta gat gtg gat gac tgt cag gat 192
Tyr Ser Asn Val Ile Phe Leu Glu Val Asp Val Asp Asp Cys Gln Asp
    50                  55                  60

gtt gct tca gag tgt gaa gtc aaa tgc atg cca aca ttc cag ttt ttt 240
Val Ala Ser Glu Cys Glu Val Lys Cys Met Pro Thr Phe Gln Phe Phe
65                  70                  75                  80

aag aag gga caa aag gtg ggt gaa ttt tct gga gcc aat aag gaa aag 288
Lys Lys Gly Gln Lys Val Gly Glu Phe Ser Gly Ala Asn Lys Glu Lys
                85                  90                  95

ctt gaa gcc acc att aat gaa tta gtc taa 318
Leu Glu Ala Thr Ile Asn Glu Leu Val

            100                 105

<210> 2
<211> 105
<212> PRT
<213> Homo sapiens

<400> 2

Met Val Lys Gln Ile Glu Ser Lys Thr Ala Phe Gln Glu Ala Leu Asp
1               5                   10                  15

Ala Ala Gly Asp Lys Leu Val Val Val Asp Phe Ser Ala Thr Trp Cys
            20                  25                  30

Gly Pro Cys Lys Met Ile Lys Pro Phe Phe His Ser Leu Ser Glu Lys
        35                  40                  45

Tyr Ser Asn Val Ile Phe Leu Glu Val Asp Val Asp Asp Cys Gln Asp
    50                  55                  60

Val Ala Ser Glu Cys Glu Val Lys Cys Met Pro Thr Phe Gln Phe Phe
65                  70                  75                  80

Lys Lys Gly Gln Lys Val Gly Glu Phe Ser Gly Ala Asn Lys Glu Lys
                85                  90                  95

Leu Glu Ala Thr Ile Asn Glu Leu Val
            100                 105

<210> 3
<211> 27
<212> DNA
<213> Homo sapiens

<400> 3
ggatccgtga agcagatcga gagcaag 27

<210> 4
<211> 30

<212>  DNA
<213>  Homo sapiens

<400>  4
gtcgacttag actaattcat taatggtggc                                        30


<210>  5
<211>  27
<212>  DNA
<213>  Homo sapiens

<400>  5
cttgatcatt ttgcaaggcc cagacca                                           27


<210>  6
<211>  27
<212>  DNA
<213>  Homo sapiens

<400>  6
tggtctgggc cttgcaaaat gatcaag                                           27


<210>  7
<211>  27
<212>  DNA
<213>  Homo sapiens

<400>  7
cttgatcatt ttggaaggcc cacacca                                           27


<210>  8
<211>  27
<212>  DNA
<213>  Homo sapiens

<400>  8
tggtgtgggc cttccaaaat gatcaag                                           27


<210>  9
<211>  27
<212>  DNA
<213>  Homo sapiens

<400>  9

tggtctgggc cttccaaaat gatcaag                                          27

<210>  10
<211>  27
<212>  DNA
<213>  Homo sapiens

<400>  10
cttgatcatt ttggaaggcc cagacca                                          27

<210>  11
<211>  318
<212>  DNA
<213>  Homo sapiens

<220>
<221>  misc_feature
<222>  (103)..(105)
<223>  nnn stands for any base for coding Lys, Asn, Arg, Ser, Thr, Ile,
       Met, Glu, Asp, Gly, Ala, Val, Gln, His, Pro, Leu, Tyr, Trp, or Ph
       e.

<220>
<221>  CDS
<222>  (1)..(318)
<223>  The 'Xaa' at location 35 stands for Lys, Asn, Arg, Ser, Thr, Ile,
       Met, Glu, Asp, Gly, Ala, Val, Gln, His, Pro, Leu, Tyr, Trp, or Ph
       e.

<400>  11
atg gtg aag cag atc gag agc aag act gct ttt cag gaa gcc ttg gac        48
Met Val Lys Gln Ile Glu Ser Lys Thr Ala Phe Gln Glu Ala Leu Asp
1               5                   10                  15

gct gca ggt gat aaa ctt gta gta gtt gac ttc tca gcc acg tgg tgt        96
Ala Ala Gly Asp Lys Leu Val Val Val Asp Phe Ser Ala Thr Trp Cys
            20                  25                  30

ggg cct nnn aaa atg atc aag cct ttc ttt cat tcc ctc tct gaa aag       144
Gly Pro Xaa Lys Met Ile Lys Pro Phe Phe His Ser Leu Ser Glu Lys
        35                  40                  45

tat tcc aac gtg ata ttc ctt gaa gta gat gtg gat gac tgt cag gat       192
Tyr Ser Asn Val Ile Phe Leu Glu Val Asp Val Asp Asp Cys Gln Asp
    50                  55                  60

gtt gct tca gag tgt gaa gtc aaa tgc atg cca aca ttc cag ttt ttt 240
Val Ala Ser Glu Cys Glu Val Lys Cys Met Pro Thr Phe Gln Phe Phe
65                  70                  75                  80

aag aag gga caa aag gtg ggt gaa ttt tct gga gcc aat aag gaa aag 288
Lys Lys Gly Gln Lys Val Gly Glu Phe Ser Gly Ala Asn Lys Glu Lys
                85                  90                  95

ctt gaa gcc acc att aat gaa tta gtc taa 318
Leu Glu Ala Thr Ile Asn Glu Leu Val
            100                 105

<210> 12
<211> 318
<212> DNA
<213> Homo sapiens

<220>
<221> CDS
<222> (1)..(318)
<223>

<400> 12
atg gtg aag cag atc gag agc aag act gct ttt cag gaa gcc ttg gac 48
Met Val Lys Gln Ile Glu Ser Lys Thr Ala Phe Gln Glu Ala Leu Asp
1               5                   10                  15

gct gca ggt gat aaa ctt gta gta gtt gac ttc tca gcc acg tgg tgt 96
Ala Ala Gly Asp Lys Leu Val Val Val Asp Phe Ser Ala Thr Trp Cys
            20                  25                  30

ggg cct tcc aaa atg atc aag cct ttc ttt cat tcc ctc tct gaa aag 144
Gly Pro Ser Lys Met Ile Lys Pro Phe Phe His Ser Leu Ser Glu Lys
        35                  40                  45

tat tcc aac gtg ata ttc ctt gaa gta gat gtg gat gac tgt cag gat 192
Tyr Ser Asn Val Ile Phe Leu Glu Val Asp Val Asp Asp Cys Gln Asp
    50                  55                  60

gtt gct tca gag tgt gaa gtc aaa tgc atg cca aca ttc cag ttt ttt 240
Val Ala Ser Glu Cys Glu Val Lys Cys Met Pro Thr Phe Gln Phe Phe
65                  70                  75                  80

aag aag gga caa aag gtg ggt gaa ttt tct gga gcc aat aag gaa aag 288
Lys Lys Gly Gln Lys Val Gly Glu Phe Ser Gly Ala Asn Lys Glu Lys
                85                  90                  95

ctt gaa gcc acc att aat gaa tta gtc taa                                    318
Leu Glu Ala Thr Ile Asn Glu Leu Val
            100                 105

<210>  13
<211>  318
<212>  DNA
<213>  Homo sapiens

<220>
<221>  CDS
<222>  (1)..(318)
<223>

<400>  13
atg gtg aag cag atc gag agc aag act gct ttt cag gaa gcc ttg gac        48
Met Val Lys Gln Ile Glu Ser Lys Thr Ala Phe Gln Glu Ala Leu Asp
1               5                   10                  15

gct gca ggt gat aaa ctt gta gta gtt gac ttc tca gcc acg tgg tct        96
Ala Ala Gly Asp Lys Leu Val Val Val Asp Phe Ser Ala Thr Trp Ser
            20                  25                  30

ggg cct tcc aaa atg atc aag cct ttc ttt cat tcc ctc tct gaa aag       144
Gly Pro Ser Lys Met Ile Lys Pro Phe Phe His Ser Leu Ser Glu Lys
        35                  40                  45

tat tcc aac gtg ata ttc ctt gaa gta gat gtg gat gac tgt cag gat       192
Tyr Ser Asn Val Ile Phe Leu Glu Val Asp Val Asp Asp Cys Gln Asp
    50                  55                  60

gtt gct tca gag tgt gaa gtc aaa tgc atg cca aca ttc cag ttt ttt       240
Val Ala Ser Glu Cys Glu Val Lys Cys Met Pro Thr Phe Gln Phe Phe
65                  70                  75                  80

aag aag gga caa aag gtg ggt gaa ttt tct gga gcc aat aag gaa aag       288
Lys Lys Gly Gln Lys Val Gly Glu Phe Ser Gly Ala Asn Lys Glu Lys
                85                  90                  95

ctt gaa gcc acc att aat gaa tta gtc taa                               318
Leu Glu Ala Thr Ile Asn Glu Leu Val
            100                 105

<210>  14
<211>  318
<212>  DNA

<213> Homo Sapiens

<220>
<221> CDS
<222> (1)..(318)
<223>

<400> 14
atg gtg aag cag atc gag agc aag act gct ttt cag gaa gcc ttg gac 48
Met Val Lys Gln Ile Glu Ser Lys Thr Ala Phe Gln Glu Ala Leu Asp
1               5                   10                  15

gct gca ggt gat aaa ctt gta gta gtt gac ttc tca gcc acg tgg tct 96
Ala Ala Gly Asp Lys Leu Val Val Val Asp Phe Ser Ala Thr Trp Ser
            20                  25                  30

ggg cct tgc aaa atg atc aag cct ttc ttt cat tcc ctc tct gaa aag 144
Gly Pro Cys Lys Met Ile Lys Pro Phe Phe His Ser Leu Ser Glu Lys
        35                  40                  45

tat tcc aac gtg ata ttc ctt gaa gta gat gtg gat gac tgt cag gat 192
Tyr Ser Asn Val Ile Phe Leu Glu Val Asp Val Asp Asp Cys Gln Asp
    50                  55                  60

gtt gct tca gag tgt gaa gtc aaa tgc atg cca aca ttc cag ttt ttt 240
Val Ala Ser Glu Cys Glu Val Lys Cys Met Pro Thr Phe Gln Phe Phe
65                  70                  75                  80

aag aag gga caa aag gtg ggt gaa ttt tct gga gcc aat aag gaa aag 288
Lys Lys Gly Gln Lys Val Gly Glu Phe Ser Gly Ala Asn Lys Glu Lys
                85                  90                  95

ctt gaa gcc acc att aat gaa tta gtc taa 318
Leu Glu Ala Thr Ile Asn Glu Leu Val
            100                 105